# EUROPÄISCHE PATENTANMELDUNG

(11) **EP 2 985 271 A1**
(43) Veröffentlichungstag der Anmeldung: **17.02.2016**
(21) Anmeldenummer: 14190091.0
(22) Anmeldetag: 23.10.2014
(51) Int. Cl.: C07C 2/78, C07C 5/48

(54) **Verfahren und Anlage zur Herstellung von Olefinen**

(30) Priorität: 11.08.2014 DE 102014111434
(71) Anmelder: Linde Aktiengesellschaft, 80331 München (DE)
(72) Erfinder: Delhomme, Clara, 81829 München (DE); Kemper, Rainer, 81375 München (DE); Meiswinkel, Andreas, 83209 Prien (DE)
(74) Vertreter: m patent group

(57) **Zusammenfassung**

Es wird ein Verfahren (100) zur Herstellung von Olefinen vorgeschlagen, bei dem in einem ersten Umsetzungsschritt (10) ein erster Produktstrom (3) gebildet wird, der Ethan und Ethylen enthält. Zumindest ein Teil des ersten Produktstroms (3) wird einem zweiten Umsetzungsschritt (20) unterworfen, in dem in dem ersten Produktstrom (3) enthaltenes Ethan unter Erhalt eines zweiten Produktstroms (6) zumindest teilweise zu Ethylen umgesetzt wird. Eine entsprechende Anlage ist ebenfalls Gegenstand der vorliegenden Erfindung.

## Beschreibung

Die Erfindung betrifft ein Verfahren und eine Anlage zur Herstellung von Kohlenwasserstoffen gemäß den Oberbegriffen der unabhängigen Patentansprüche.

### Stand der Technik

Zur Herstellung von niedrigen Olefinen, insbesondere von Ethylen und Propylen, werden derzeit nahezu ausschließlich das Steamcracking von Kohlenwasserstoffen sowie das katalytische Cracken in Raffinerieanlagen verwendet. Als Einsatz (Feed) werden hierbei sowohl Ethan und Propan (insbesondere zur Herstellung der Produkte Ethylen bzw. Propylen) als auch höhere Kohlenwasserstoffe (Butan, Butan-Propan-Gemische, Naphtha etc.) eingesetzt. Steamcracking ist jedoch ein ausgesprochen energieintensiver Prozess und führt u.a. zur Bildung einer Reihe von Nebenprodukten, die eine aufwendige Abtrennung und Aufreinigung der Produktströme erfordern.

Alternativ wurde in der Historie Ethylen auch durch die Dehydratisierung von Ethanol hergestellt. Diese Technologie erlebt aktuell einen Aufschwung, z.B. in Brasilien durch die lokale Verfügbarkeit von kostengünstigem Ethanol in großen Mengen.

Ferner existieren kommerzielle Technologien zur selektiven Umwandlung von Propan in Propylen durch die Propandehydrierung (PDH). Auch die Konversion von Ethylen und 2-Buten zu Propylen bzw. umgekehrt (sogenannte Olefin-Metathese) ist bekannt und wird in mehreren Anlagen weltweit großtechnisch praktiziert.

Ein unmittelbares Konkurrenzverfahren zum Steamcracking könnte in der Zukunft auch die oxidative Dehydrierung (ODH) sein, mittels derer sich ebenfalls Paraffine, insbesondere Ethan, über einen Katalysator selektiv in die korrespondierenden Olefine umwandeln lassen. Katalysatoren für die Umsetzung von Ethan zu Ethylen haben in der letzten Zeit eine deutliche Verbesserung erfahren, wie beispielsweise in der europäischen Patentanmeldung Nr. 13004750.9 offenbart.

Weitere Verfahren zur direkten Nutzung von Methan, das in großen Mengen als Bestandteil von Erdgas verfügbar ist, und insbesondere zur gezielten Darstellung von höheren Kohlenwasserstoffen aus Methan, befinden sich derzeit in intensiver Entwicklung. Aktueller Stand ist hier entweder die Umsetzung zu Synthesegas und anschließende weitere Schritte, wie z.B. Fischer-Tropsch-Synthese, oder Routen über Oxygenate als Intermediat, wie z.B. Methanol. In letzter Zeit zeigen auch Entwicklungsarbeiten im Bereich der direkten Umsetzung von Methan in oxidativen, heterogen katalysierten Verfahren zu höheren Kohlenwasserstoffen erste Erfolge (sogenannte oxidative Kopplung von Methan, OCM). Entsprechende Verfahren erscheinen insbesondere zur Herstellung von C2-Produkten besonders vielversprechend und laufen entsprechend der nachfolgend erläuterten Reaktionsschemata ab:

2 CH₄ + 0,5 O₂ → C₂H₆ + H₂O ΔH_{R} = -177 kJ/mol

2 CH₄ + O₂ → C₂H₄ + 2 H₂O ΔH_{R} = -282 kJ/mol

Die genannten Reaktionen sind jedoch zumeist in der Selektivität zu einem bestimmten Produkt und/oder in ihrem Umsatz limitiert. Letzteres ist insbesondere darauf zurückzuführen, dass bei stöchiometrischer Sauerstoffzugabe Nebenreaktionen, die zur Totaloxidation mit Bildung von unerwünschtem Kohlenmonoxid und Kohlendioxid führen, energetisch begünstigt sind und daher stark zunehmen. Sauerstoff wird daher in unterstöchiometrischer Menge zugegeben, so dass zwangsläufig eine beträchtliche Menge des eingesetzten Methans nicht umgesetzt werden kann. Typischerweise werden in der OCM Konversionsraten von Methan unterhalb von 50% verwendet, um akzeptable C2-Selektivitäten aufrechtzuerhalten. Die Rückführung nicht umgesetzten Methans erfordert eine Bearbeitung großer Recyclevolumina und eine beträchtliche Verdichterleistung, da Methan typischerweise unter Druck umgesetzt wird.

Bei der OCM, die typischerweise bei Temperaturen oberhalb von 500 °C stattfindet, wird zwar ein erheblicher Anteil an C2-Kohlenwasserstoffen gebildet (diese stellen zumeist den Hauptanteil der gebildeten höheren Kohlenwasserstoffe), jedoch ist ein gravierender Nachteil insbesondere der, dass der Ethylenanteil in dieser C2-Fraktion beschränkt und zudem signifikante Mengen an Ethan darin enthalten sind. Somit ist eine aufwendige Reinigung und Auftrennung des Produktgases erforderlich. Dabei ist die Trennung von Ethan und Ethylen aufgrund ihrer geringen Siedepunktsdifferenz technisch aufwendig und erfordert einen entsprechend hohen Energieeinsatz. Diese Trennung erfolgt typischerweise in einem sogenannten C2-Splitter bei ca. 7 bis 25 bar und Temperaturen deutlich unterhalb von 0 °C (kryogene Trennung).

Entsprechend dem Stand der Technik sind also, zusammengefasst, OCM-Verfahren hinsichtlich ihres Umsatzes beschränkt, was zu einer großen zu behandelnden Methanmenge führt. Bei der gebildeten C2-Fraktion handelt es sich um eine Mischung aus Ethan und Ethylen, was eine aufwendige Trennung erfordert.

Aufgabe der vorliegenden Erfindung ist es daher, die Ausbeute an Ethylen bei der OCM zu erhöhen und insbesondere die Schritte zur Gewinnung und Aufreinigung von Ethylen zu vereinfachen. Verallgemeinert gesprochen besteht die Aufgabe der vorliegenden Erfindung darin, ein Gasgemisch, das einem in der OCM produzierten Gasgemisch entspricht, weiter aufzubereiten, um Wertprodukte, insbesondere Ethylen, auf einfachere und effizientere Weise gewinnen zu können. Die Erfindung soll explizit nicht auf die Bearbeitung von Gasgemischen aus der OCM beschränkt sein, sondern erstreckt sich auch auf andere vergleichbare Gasgemische, die eine Mischung von Ethan und Ethylen beinhalten.

### Offenbarung der Erfindung

Diese Aufgabe wird durch ein Verfahren und eine Anlage zur Herstellung von Kohlenwasserstoffen mit den Merkmalen der unabhängigen Patentansprüche gelöst. Bevorzugte Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Zu den nachfolgend verwendeten Begriffen und zu technischen Details der eingesetzten Verfahren sei auf einschlägige Fachliteratur verwiesen. Zum Steamcracking wird insbesondere auf Zimmermann, H. und Walzl, R.: Ethylene, In: Ullmann's Encyclopedia of Industrial Chemistry, Weinheim: Wiley-VCH, Onlinepublikation 2007, DOI: 10.1002/14356007.a10_045.pub2, sowie Irion, W.W. und Neuwirth, O.S.: Oil Refining, In: Ullmann's Encyclopedia of Industrial Chemistry, Weinheim: Wiley-VCH, Onlinepublikation 2000, DOI: 10.1002/14356007.a18_051, zur OCM auf Zavyalova, U. et al.: Statistical Analysis of Past Catalytic Data on Oxidative Methane Coupling for New Insights into the Composition of High-Performance Catalysts, ChemCatChem 3, 2011, 1935-1947 und zur ODH auf Cavani, F. et al.: Oxidative Dehydrogenation of Ethane and Propane: How far from commercial implementation?, Catal. Today 127, 2007, 113-131, Botella, P. et al.: Selective Oxidative Dehydrogenation of Ethane on MoVTeNbO Mixed Metal Oxide Catalysts, J. Catal. 225, 2004, 428-438, Ivars, F. et al.: Selective Oxidation of Short-Chain Alkanes over Hydrothermally Prepared MoVTeNbO Catalysts, Top. Catal. 38, 2006, 59-67, López Nieto, J.M. et al.: Oxidative Dehydrogenation of Ethane on Te-containing MoVNbO Catalysts, Catal. Today 91-92, 2004, 241-245 sowie López Nieto, J.M. et al.: Reaction Products and Pathways in the Selective Oxidation of C2-C4 Alkanes on MoVTeNb Mixed Oxide Catalysts, Catal. Today 157, 2010, 291-296 hingewiesen. Ein aktueller Überblick zur ODH mit verschiedenen Katalysatorsystemen findet sich in Gärtner, C. et al.: Oxidative Dehydrogenation of Ethane: Common Principles and Mechanistic Aspects, ChemCatChem 5, 2013, 3196-3217.

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Ein "Produktstrom", "Produktgemisch" oder "Produktgas" ist ein Gemisch an Kohlenwasserstoffen und anderen Verbindungen, das aus einem Verfahren wie Steamcracking, OCM oder ODH erhalten wird. Ein entsprechendes Gemisch kann in dem Verfahren gebildete Verbindungen, aber auch in dem Verfahren nicht umgesetzte Verbindungen des Einsatzes enthalten.

Im Rahmen der vorliegenden Erfindung können absorptive, adsorptive und/oder destillative Verfahren zur Abtrennung von Komponenten aus entsprechenden Produktströmen zum Einsatz kommen. Zur destillativen bzw. absorptiven Trennung können insbesondere Destillationssäulen und Absorptionskolonnen verwendet werden, die insbesondere bei kryogenen Temperaturen arbeiten. Zur Auslegung und Ausgestaltung entsprechender Vorrichtungen sei auf einschlägige Lehrbücher verwiesen (siehe beispielsweise Sattler, K.: Thermische Trennverfahren. Grundlagen, Auslegung, Apparate. Weinheim: Wiley-VCH, 3. Auflage 2001).

Adsorptive Verfahren können insbesondere unter Verwendung eines oder mehrerer Adsorptionsbehälter durchgeführt werden, die ein geeignetes Adsorptionsmaterial, beispielsweise Molekularsieb, enthalten. Adsorptive Verfahren sind grundsätzlich bekannt und eignen sich insbesondere zur Abtrennung geringerer Mengen an Komponenten in entsprechenden Gemischen.

### Vorteile der Erfindung

Im Rahmen der vorliegenden Erfindung wird beispielsweise die Ethylenausbeute einer OCM dadurch erhöht, dass der erhaltene Produktstrom unmittelbar einer weiteren Reaktion zugeführt wird, bei der Ethan selektiv in Ethylen überführt wird. Neben Produktströmen aus der OCM können aber auch weitere Produktströme mit entsprechender Zusammensetzung (insbesondere gekennzeichnet durch einen nennenswerten Ethananteil) entsprechend bearbeitet werden. Als weitere Reaktion kommt vorzugsweise die ODH zum Einsatz, die unter Zugabe eines Oxidationsmittels, insbesondere Sauerstoff, selektiv Ethan zu Ethylen umwandelt. Dabei ist eine zusätzliche Einspeisung des Oxidationsmittels stromauf der ODH möglich.

Besonders bevorzugt wird dabei ein Katalysator verwendet, wie er beispielsweise in der europäischen Patentanmeldung Nr. 13004750.9 offenbart und unten nochmals im Detail erläutert ist. Grundsätzlich kommen aber alle geeigneten Katalysatoren in Frage, die eine ODH mit ausreichendem Umsatz und ausreichender Selektivität erlauben.

Die vorliegende Erfindung schlägt ein Verfahren zur Herstellung von Olefinen vor, bei dem in einem ersten Umsetzungsschritt ein erster Produktstrom gebildet wird, der Ethan und Ethylen enthält. Wie bereits zuvor erläutert, kann es sich bei dem ersten Umsetzungsschritt insbesondere um eine OCM handeln, es sind jedoch auch andere Verfahren denkbar, in denen Produktströme bereitgestellt werden, die zumindest Ethan und Ethylen enthalten, und aus denen Ethylen abgetrennt bzw. in erhöhter Menge erhalten werden soll. Insbesondere können entsprechende Produktströme neben Ethan und Ethylen auch Methan und höhere Kohlenwasserstoffe enthalten. Wie erläutert, enthält ein entsprechender Produktstrom aus der OCM typischerweise vergleichsweise große Mengen an Methan, beispielsweise über 50%. Der erste Umsetzungsschritt kann dabei auch mehrere chemische Reaktionen umfassen, beispielsweise kann die OCM mit Steamcracking gekoppelt werden.

Erfindungsgemäß ist vorgesehen, dass zumindest ein Teil des ersten Produktstroms einem zweiten Umsetzungsschritt unterworfen wird, wie erläutert vorzugsweise der ODH, in welchem in dem ersten Produktstrom enthaltenes Ethan unter Erhalt eines zweiten Produktstroms zumindest teilweise zu Ethylen umgesetzt wird. Die vorliegende Erfindung schlägt also vor, wie bereits erläutert, in einem entsprechenden (ersten) Produktstrom enthaltenes, als Produkt unerwünschtes Ethan weiter zu Ethylen umzusetzen, so dass die Gesamtausbeute des Verfahrens und damit letztlich die Ausbeute an Ethylen des ersten Umsetzungsschritts weiter erhöht werden kann.

Durch die vorliegende Erfindung kann insbesondere die Ethylenausbeute im Produktstrom einer OCM deutlich erhöht werden. Dies ermöglicht eine Vereinfachung der nachgeschalteten Produktaufreinigung, da eine geringere Menge an Ethan von Ethylen abzutrennen ist. Zudem kann der nachgeschaltete zweite Umsetzungsschritt, beispielsweise die ODH, die in dem ersten Produktstrom enthaltene fühlbare Wärme nutzen. Da die OCM typischerweise bei höheren Temperaturen erfolgt als die ODH, kann die Reaktionswärme aus der OCM zudem zur Vorwärmung, beispielsweise von Feedströmen für die ODH, also den zweiten Umsetzungsschritt, oder die Erzeugung von Wasserdampf als Verdünnungsmedium für die ODH, genutzt werden. Somit ergibt sich auch ein weiterer Vorteil aus einer Energieintegration und -nutzung.

Eine ODH wird insbesondere unter Verwendung eines Metalloxidkatalysators durchgeführt, der zumindest ein Element aus der Gruppe Mo, Te, Nb, V, Cr, Dy, Ga, Sb, Ni, Co, Pt und Ce enthält. Wie bereits in der zuvor zitierten europäischen Patentanmeldung 13004750.9 offenbart, eignen sich derartige Katalysatoren insbesondere für die Verwendung in entsprechenden Verfahren. Besonders bevorzugt sind Metalloxidkatalysatoren der allgemeinen Zusammensetzung MoVTeNbOx, die einen besonders hohen Anteil an M1-Phase enthalten. Diese M1-Phase kann durch entsprechende Herstellung, insbesondere durch eine Behandlung mit Wasserdampf und/oder Sauerstoff, maximiert werden. Zu Details sei auch auf die oben zitierte Literatur verwiesen.

In einem entsprechenden Verfahren ist es besonders vorteilhaft, wenn der erste Umsetzungsschritt und der zweite Umsetzungsschritt in einem eine bauliche Einheit bildenden Reaktor mit Reaktionszonen durchgeführt wird, die jeweils zur Durchführung des ersten Umsetzungsschritts und des zweiten Umsetzungsschritts eingerichtet sind. Ein entsprechender Reaktor kann beispielsweise einen zwischengeschalteten Wärmetauscher und zusätzliche Einspeisemöglichkeiten für Oxidationsmittel, Recycleströme und/oder Verdünnungsmedien aufweisen. Innerhalb eines entsprechenden Reaktors können die unterschiedlichen Reaktionszonen mit geeigneten Katalysatorfüllungen bereitgestellt werden. Vorzugsweise werden in dieser Ausgestaltung Katalysatoren verwendet, deren thermische Betriebsfenster möglichst nahe beieinander liegen. Auf diese Weise wird eine weitgehende Energieintegration und eine besonders kompakt zu bauende Anlage ermöglicht.

Besonders vorteilhaft ist es, wenn aus dem zweiten Produktstrom Methan abgetrennt wird, das, wie erwähnt, insbesondere dann wenn der erste Umsetzungsschritt eine OCM umfasst, in vergleichsweise großer Menge gebildet und in die OCM zurückgeführt werden kann. Die Abtrennung erfolgt jedoch nicht direkt stromab der OCM sondern erst stromab des zweiten Umsetzungsschritts, so muss also nur an einer Stelle eine Trennsequenz durchlaufen werden. Im Gegensatz zur Trennung der C2-Kohlenwasserstoffe Ethan und Ethylen erweist sich eine Abtrennung von Methan aufgrund der höheren Siedepunktsdifferenz als deutlich einfacher.

Enthält aufgrund einer nicht vollständigen Umsetzung in dem zweiten Umsetzungsschritt der zweite Produktstrom noch Ethan, wird dieses vorzugsweise aus dem zweiten Produktstrom abgetrennt. Da Ethan jedoch aufgrund der zumindest teilweisen Umsetzung in dem zweiten Umsetzungsschritt nun in deutlich geringerer Menge vorliegt, als dies bei Verfahren der Fall ist, die lediglich den ersten Umsetzungsschritt umfassen, gestaltet sich eine entsprechende Abtrennung als sehr viel einfacher. Im Gegensatz zu herkömmlicherweise eingesetzten C2-Splittern, also Destillationssäulen, die mit kryogen verflüssigten Gasgemischen betrieben werden, kann zur Abtrennung von Ethan gegebenenfalls auch ein absorptives oder adsorptives Verfahren, beispielsweise unter Verwendung einer Absorptionskolonne oder von geeigneten Adsorbermaterialien, eingesetzt werden. In besonderer Weise eignen sich auch Membranverfahren, wie sie ebenfalls grundsätzlich bekannt sind. In jedem Fall wird das aus dem zweiten Produktstrom abgetrennte Ethan vorzugsweise erneut dem zweiten Umsetzungsschritt unterworfen, so dass dieses Ethan zunehmend zu Ethylen umgesetzt werden kann.

Es wird also vorteilhafterweise das Ethan aus einem noch zumindest Ethan und Ethylen enthaltenden, aus dem zweiten Produktstrom gebildeten Gasgemisch (beispielsweise nach Abtrennung von Methan) absorptiv, adsorptiv und/oder destillativ abgetrennt. Wie erläutert, erweist sich eine derartige Abtrennung als besonders einfach, weil keine großen Ethanmengen mehr zu bewältigen sind.

Es ist besonders vorteilhaft, wenn in dem zweiten Umsetzungsschritt ein in dem zweiten Umsetzungsschritt nicht umgesetztes inertes Verdünnungsmittel zugegeben wird, das zumindest teilweise in den zweiten Produktstrom übergeht. Eine derartige Zugabe erfolgt beispielsweise durch Zuspeisen zu einem entsprechenden ersten Produktstrom, beispielsweise in Form eines Recyclestroms und/oder eines Frischeinsatzes eines von extern zugeführten Verdünnungsmittels. Als Verdünnungsmittel kommen prinzipiell alle geeigneten inerten Medien, insbesondere aber Wasserdampf, Stickstoff, Helium, Argon, Kohlendioxid oder eine Kombination hiervon in Frage. Insbesondere Wasserdampf kann beispielsweise durch Abwärme des ersten Umsetzungsschritts, beispielsweise, wie erläutert, der OCM, erzeugt werden. Auch Stickstoff aus Luft kann als Verdünnungsmittel zugeführt werden, wobei der Sauerstoff der Luft als Oxidationsmittel (siehe unten) dient.

Vorzugsweise wird das Verdünnungsmittel aus dem zweiten Produktstrom abgetrennt und zumindest teilweise erneut in dem zweiten Umsetzungsschritt zugegeben. Entsprechendes Verdünnungsmittel geht also dem Prozess nicht verloren, sondern kann vorteilhafterweise einfach und energiesparend rezykliert werden.

In einem entsprechenden Verfahren kann vorzugsweise auch ein Teil des ersten Produktstroms abgekühlt werden. Um die Temperatur des Produktstroms beispielsweise aus einer OCM (mit typischerweise mehr als 500 °C) an die ODH (die bei typischerweise weniger als 500 °C abläuft) anzupassen, kann ein Wärmetauscher zwischengeschaltet werden, um z.B. einen der Recycleströme oder auch ein von außen zugeführtes Inertmedium oder Oxidationsmittel, wie z.B. Luft, Sauerstoff oder sauerstoffangereicherte Luft, anzuwärmen. Ferner ist auch insbesondere die Erzeugung von Dampf denkbar, der entweder im Verfahren selbst als Inertmedium zugeführt oder aber auch für andere Zwecke verwendet werden kann.

Mit besonderem Vorteil wird in dem ersten und in dem zweiten Umsetzungsschritt zumindest ein sauerstoffhaltiges Gasgemisch eingesetzt. In dem ersten Umsetzungsschritt handelt es sich dabei typischerweise um reinen Sauerstoff oder um ein sauerstoffreiches Gemisch bzw. Luft, die den zur OCM erforderlichen Sauerstoff bereitstellt. In dem zweiten Umsetzungsschritt kann beispielsweise in einer ODH entsprechender Sauerstoff verwendet werden. Bei Verwendung eines geeigneten Katalysators in dem zweiten Umsetzungsschritt und bei geeigneten Reaktionsbedingungen kann hier auch in dem ersten Umsetzungsschritt, beispielsweise in der OCM, gebildetes Kohlendioxid als Oxidationsmittel genutzt werden. Auf die Verwendung von Luft wurde bereits oben hingewiesen.

Eine Anlage zur Herstellung von Olefinen, die dafür eingerichtet ist, in einem ersten Umsetzungsschritt einen ersten Produktstrom zu bilden, der Ethan und Ethylen enthält, ist ebenfalls Gegenstand der vorliegenden Erfindung. Die Anlage weist erfindungsgemäß Mittel auf, die dafür eingerichtet sind, zumindest einen Teil des ersten Produktstroms einem zweiten Umsetzungsschritt zu unterwerfen, in dem in dem ersten Produktstrom enthaltenes Ethan unter Erhalt eines zweiten Produktstroms zumindest teilweise zu Ethylen umgesetzt wird.

Eine derartige Anlage ist insbesondere zur Durchführung eines Verfahrens eingerichtet, wie es zuvor erläutert wurde. Auf die erläuterten Merkmale und Vorteile wird daher ausdrücklich verwiesen.

Die Erfindung wird unter Bezugnahme auf die beigefügte Zeichnung näher erläutert, die eine bevorzugte Ausführungsform der Erfindung zeigt.

### Kurze Beschreibung der Zeichnung

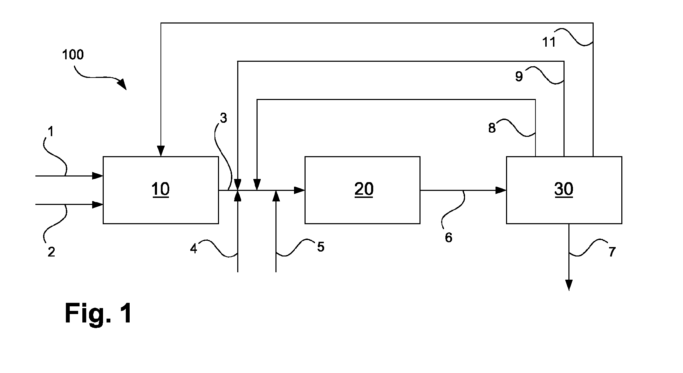

Figur 1 zeigt ein Verfahren gemäß einer Ausführungsform der Erfindung in schematischer Darstellung.

### Ausführungsform der Erfindung

In Figur 1 ist ein Verfahren gemäß einer Ausführungsform der Erfindung schematisch dargestellt und insgesamt mit 100 bezeichnet. Die Figur 1 veranschaulicht gleichzeitig die Grundelemente einer entsprechenden Anlage.

Das in Figur 1 dargestellte Verfahren 100 umfasst einen ersten Umsetzungsschritt 10 und einen zweiten Umsetzungsschritt 20 sowie einen Trennschritt 30. Der erste Umsetzungsschritt 10 kann insbesondere eine oxidative Kopplung von Methan (OCM), gegebenenfalls mit nachgeordneten Verfahrensschritten, umfassen, der zweite Umsetzungsschritt 20 umfasst insbesondere eine oxidative Dehydrierung (ODH). Der Trennschritt 30 kann insbesondere unter Verwendung wenigstens einer destillativen Trenneinheit realisiert sein, die insbesondere kryogen ausgeführt sein kann, in bestimmten Fällen kann auf Trenneinheiten wie Absorptionskolonnen und Destillationssäulen - insbesondere in kryogener Ausführung -jedoch auch verzichtet werden. In diesem Fall können insbesondere adsorptive, absorptive und/oder Membranverfahren zur Trennung in dem Trennschritt 30 eingesetzt werden. Beliebige Kombinationen entsprechender Verfahren, beispielsweise mit destillativen und kryogenen Trennschritten, sind möglich.

Dem ersten Umsetzungsschritt 10 werden im dargestellten Beispiel zwei Einsatzströme 1 und 2 zugeführt, wobei es sich bei dem Einsatzstrom 1 insbesondere um einen methanreichen Strom, beispielsweise reines Methan, Erdgas oder eine aus Erdgas gewonnene Fraktion, handeln kann. Der Einsatzstrom 2 umfasst insbesondere Sauerstoff und/oder Luft. In dem ersten Umsetzungsschritt 10, der hier eine OCM umfasst, wird Methan in dem Einsatzstrom 1 mit Sauerstoff in dem Einsatzstrom 2 zumindest teilweise katalytisch zu Ethan und Ethylen sowie weiteren Produkten umgesetzt, die sich in einem ersten Produktstrom 3 wiederfinden. Vor dem zweiten Umsetzungsschritt 20, der im dargestellten Beispiel eine ODH umfasst, kann zu dem ersten Produktstrom 3 oder einem entsprechenden Teil hiervon, der dem zweiten Umsetzungsschritt 20 unterworfen wird, beispielsweise ein Verdünnungsmittel 4, beispielsweise Wasserdampf oder ein Inertgas, sowie ein sauerstoffhaltiger Strom 5, beispielsweise Sauerstoff oder Luft, zugegeben werden.

In dem zweiten Umsetzungsschritt 20 wird ein zweiter Produktstrom 6 erhalten, der zumindest teilweise dem Trennschritt 30 unterworfen wird. In dem Trennschritt 30 wird Ethylen 7 abgetrennt. Je nach Zusammensetzung des zweiten Produktstroms 6 können hier auch weitere Ströme abgetrennt werden, beispielsweise Ethan 8, ein Verdünnungsmittel 9 und/oder Methan 10. Wird Ethan 8 abgetrennt, wird dieses vorzugsweise stromauf des zweiten Umsetzungsschritts 20, der hier, wie erläutert, eine ODH umfasst, mit dem ersten Produktstrom 3 vereinigt. Das Verdünnungsmittel 9 wird ebenfalls an dieser Stelle rückgeführt. Eine Rückführung von Methan 10 erfolgt hingegen vorzugsweise in den ersten Umsetzungsschritt, der hier eine OCM umfasst.

Wie aus der Figur 1 ersichtlich, ergibt sich durch ein erfindungsgemäßes Verfahren eine weitgehende Integration entsprechender Recycleströme, als Produkt wird lediglich noch Ethylen bereitgestellt. Dieses kann aufgrund der geringeren Gehalte von insbesondere Ethan in dem zweiten Produktstrom 6 vereinfacht gewonnen und in hoher Reinheit bereitgestellt werden.

## Patentansprüche

1. Verfahren (100) zur Herstellung von Olefinen, bei dem in einem ersten Umsetzungsschritt (10) ein erster Produktstrom (3) gebildet wird, der Ethan und Ethylen enthält, **dadurch gekennzeichnet, dass** zumindest ein Teil des ersten Produktstroms (3) einem zweiten Umsetzungsschritt (20) unterworfen wird, in dem in dem ersten Produktstrom (3) enthaltenes Ethan unter Erhalt eines zweiten Produktstroms (6) zumindest teilweise zu Ethylen umgesetzt wird.

2. Verfahren (100) nach Anspruch 1, wobei der erste Umsetzungsschritt (10) eine oxidative Kopplung von Methan umfasst.

3. Verfahren (100) nach Anspruch 1 oder 2, wobei der zweite Umsetzungsschritt (20) eine oxidative Dehydrierung umfasst.

4. Verfahren nach Anspruch 3, bei dem die oxidative Dehydrierung unter Verwendung eines Metalloxidkatalysators durchgeführt wird, der zumindest ein Element aus der Gruppe Mo, Te, Nb, V, Cr, Dy, Ga, Sb, Ni, Co, Pt und Ce enthält.

5. Verfahren nach einem der vorstehenden Ansprüche, wobei der erste Umsetzungsschritt (10) und der zweite Umsetzungsschritt (20) in einem eine bauliche Einheit bildenden Reaktor mit Reaktionszonen durchgeführt wird, die zur Durchführung des ersten Umsetzungsschritts (10) und des zweiten Umsetzungsschritts (20) eingerichtet sind.

6. Verfahren nach einem der vorstehenden Ansprüche, wobei aus dem zweiten Produktstrom (6) Methan abgetrennt wird.

7. Verfahren nach Anspruch 6, wobei das aus dem zweiten Produktstrom (6) abgetrennte Methan in den ersten Umsetzungsschritt (10) zurückgeführt wird.

8. Verfahren nach einem der vorstehenden Ansprüche, wobei aus dem zweiten Produktstrom (6) Ethan abgetrennt wird.

9. Verfahren nach Anspruch 8, wobei das aus dem zweiten Produktstrom abgetrennte Ethan in den zweiten Umsetzungsschritt (20) zurückgeführt wird.

10. Verfahren nach Anspruch 8 oder 9, bei dem das Ethan aus einem noch zumindest Ethan und Ethylen enthaltenden, aus dem zweiten Produktstrom (6) gebildeten Gasgemisch absorptiv, adsorptiv und/oder destillativ abgetrennt wird.

11. Verfahren nach einem der vorstehenden Ansprüche, wobei in dem zweiten Umsetzungsschritt (20) ein in dem zweiten Umsetzungsschritt (20) nicht umgesetztes inertes Verdünnungsmittel (4, 9) zugegeben wird, das zumindest teilweise in den zweiten Produktstrom (6) übergeht.

12. Verfahren nach Anspruch 11, wobei das Verdünnungsmittel (4, 9) aus dem zweiten Produktstrom (6) abgetrennt und zumindest teilweise erneut in den zweiten Umsetzungsschritt (20) zurückgeführt wird.

13. Verfahren nach einem der vorstehenden Ansprüche, wobei zumindest ein Teil des ersten Produktstroms (3) abgekühlt wird.

14. Verfahren nach einem der vorstehenden Ansprüche, bei dem in dem ersten und dem zweiten Umsetzungsschritt (10, 20) zumindest ein sauerstoffhaltiges Gasgemisch eingesetzt wird.

15. Anlage zur Herstellung von Olefinen, die dafür eingerichtet ist, in einem ersten Umsetzungsschritt (10) einen ersten Produktstrom (3) zu bilden, der Ethan und Ethylen enthält, **dadurch gekennzeichnet, dass** die Anlage Mittel aufweist, die dafür eingerichtet sind, zumindest einen Teil des ersten Produktstroms (3) einem zweiten Umsetzungsschritt (20) zu unterwerfen, in dem in dem ersten Produktstrom (3) enthaltenes Ethan unter Erhalt eines zweiten Produktstroms (6) zumindest teilweise zu Ethylen umgesetzt wird.

16. Anlage nach Anspruch 15, die zur Durchführung eines Verfahrens nach einem der Ansprüche 1 bis 14 eingerichtet ist.
